# EUROPEAN PATENT APPLICATION

(11) **EP 2 638 886 A1**
(43) Date of publication of application: **18.09.2013**
(21) Application number: 11840139.7
(22) Date of filing: 30.09.2011
(51) Int. Cl.: A61F 7/10, A47G 9/10

(54) **HEAD-COOLING PILLOW AND HEAD-COOLING DEVICE**

(30) Priority: 12.11.2010 JP 2010254149
(71) Applicant: Light Optical Works, Ltd., Suwa-City, Nagano 392-0015 (JP)
(72) Inventor: IWANAMI, Masatomi, Suwa-City Nagano 392-0015 (JP); URAI, Katsuji, Suwa-City Nagano 392-0015 (JP)
(74) Representative: Klunker . Schmitt-Nilson . Hirsch
(86) International application number: PCT/JP2011/005529
(87) International publication number: WO 2012/063400

(57) **Abstract**

[Problems to be solved]

To provide a head cooling pillow which is capable of enhancing cooling efficiency for the head, suppressing pressure rise in the inside of the pillow, and improving placing feeling of the head on the pillow.

[Means to solve the Problems]

The head cooling pillow 2 includes a pillow main body 11 which is formed in a bag shape and is structured so that refrigerant is passed through its inside, a partition member 12b which is disposed in the inside of the pillow main body 11 for forming the flow passage 13 through which the refrigerant is passed, and a support member 12c structure to support the head of a user. In the head cooling pillow 2, the head is cooled by the refrigerant passing through the flow passage 13.

## Description

### [Technical Field]

The present invention relates to a head cooling pillow structured to cool the head of a user and relates to a head cooling device including the head cooling pillow.

### [Background Art]

Conventionally, an affected part cooling device structured to cool the head of a patient has been proposed (see, for example, Patent Literature 1). The affected part cooling device described in Patent Literature 1 includes an affected part cooling body (pillow) which is formed in a pillow shape and a cooling device structured to cool the pillow. The pillow includes a bag-shaped main body which is formed of rubber or the like and liquid-state silicon resin which is filled into the main body. A refrigerant pipe is disposed in the inside of the pillow so as to pass through the inside of the liquid silicon resin. Further, the cooling device includes a heat exchanger structured to cool refrigerant passing through the inside of the refrigerant pipe and a pump structured to feed the refrigerant into the refrigerant pipe. In the affected part cooling device, the liquid-state silicon resin is cooled by the refrigerant passing through the refrigerant pipe and the head of a patient is cooled by the cooled liquid-state silicon resin.

Further, in Patent Literature 1, a second affected part cooling device is also disclosed in addition to the above-mentioned affected part cooling device. In the second affected part cooling device, a meandering groove is formed on divided faces of two divided bodies which are formed of synthetic resin having flexibility and the two divided bodies are joined to each other to structure an affected part cooling body (pillow). In the second affected part cooling device, refrigerant is supplied from the cooling device to a flow passage formed by the meandering groove and the head is cooled by the refrigerant passing through the flow passage.

Further, conventionally, a continuous water cooler for cooling the head of a patient has been also proposed (see, for example, Patent Literature 2). Partition plates forming a water passage in the inside of the pillow are disposed in the continuous water cooler described in Patent Literature 2. Water is supplied to the water passage of the inside of the pillow from a faucet of water service through a hose. Further, in the continuous water cooler, the head is cooled by the water passing through the water passage.

### [Citation List]

### [Patent Literature]

[PTL 1] Japanese Patent Laid-Open No. Hei 8-299379
[PTL 2] Japanese Patent Laid-Open No. 2000-139995

### [Summary of Invention]

### [Technical Problem]

In the affected part cooling device described in Patent Literature 1, the liquid-state silicon resin is cooled by the refrigerant passing through the refrigerant pipe to cool the head of a patient by the cooled liquid-state silicon resin. In other words, in the affected part cooling device, the head is cooled through two times of heat exchange and thus cooling efficiency for the head is lowered. On the other hand, in the second affected part cooling device described in Patent Literature 1, the head of a patient is cooled by the refrigerant passing through the flow passage which is formed of the meandering groove in the inside of the pillow. In other words, in the second affected part cooling device, the head is cooled by one time of heat exchange and thus cooling efficiency of the head can be enhanced. However, in the second affected part cooling device, the pillow is formed of synthetic resin having flexibility and thus, when the head is placed on the pillow, the pressure in the inside of the pillow (in other words, pressure in the inside of the flow passage) becomes higher. Therefore, in the second affected part cooling device, the pressure resistances of the heat exchanger and the pump in the cooling device are required to be enhanced and thus the size of the cooling device may be increased.

On the other hand, in the continuous water cooler described in Patent Literature 2, the head is cooled by the water passing through the water passage which is formed by the partition plates in the inside of the pillow and the head is cooled by one time of heat exchange. Therefore, the cooling efficiency for the head can be enhanced in the continuous water cooler. Further, in the continuous water cooler, when the head is placed on the pillow, the head is supported by the partition plates and thus the pressure rise in the inside of the pillow can be suppressed. Therefore, in a case that the heat exchanger and the pump are connected with the pillow, a small heat exchanger and a small pump having a low degree of pressure resistance may be used. However, in the continuous water cooler, the head is supported by the thin plate-shaped partition plates and thus placing feeling of the head on the pillow is not good and an uncomfortable feeling is given to the user.

In view of the problem described above, an objective of the present invention is to provide a head cooling pillow which is capable of enhancing cooling efficiency for the head, suppressing the pressure rise in the inside of the pillow, and improving placing feeling of the head on the pillow, and to provide a head cooling device including the head cooling pillow.

### [Solution to Problem]

In order to attain the first objective, the present invention provides a head cooling pillow including a pillow main body which is formed in a bag shape and is structured so that refrigerant is passed through its inside, a partition member which is disposed in an inside of the pillow main body for forming an flow passage through which the refrigerant is passed, and a support member structured to support a head of a user. The head is cooled by the refrigerant passing through the flow passage.

In the head cooling pillow in accordance with the present invention, a flow passage is formed in the inside of a pillow main body by utilizing a partition member which is disposed in the inside of the pillow main body and the head is cooled by refrigerant passing through the flow passage. Therefore, in the present invention, the head is cooled by one time of heat exchange and, as a result, cooling efficiency for the head is enhanced. Further, according to the head cooling pillow in the present invention, a support member and a partition member structured to support the head are provided and thus, when the head is placed on the head cooling pillow, the head is supported by the support member and the partition member. Therefore, according to the present invention, the pressure rise in the inside of the head cooling pillow is suppressed. Further, in accordance with the present invention, the support member structured to support the head is provided and thus the head placed on the head cooling pillow is supported by the support member in addition to the partition member. Therefore, according to the present invention, placing feeling of the head on the head cooling pillow is improved.

In the present invention, the support member is, for example, a plurality of support projections. In this case, the structure of the support member is simplified.

In the present invention, a plurality of the support projections is, for example, disposed in the inside of the flow passage of the pillow main body. In this case, the pressure rise in the inside of the head cooling pillow is effectively suppressed by a plurality of the support projections and the partition member which are disposed in the inside of the pillow main body.

In the present invention, it is preferable that a height of the partition member and a height of the support projection in a thickness direction of the head cooling pillow are substantially equal to each other. According to this structure, when the head is placed on the head cooling pillow, the partition member and the support projection are abutted with an inner wall of the pillow main body with a certain predetermined pressure. Therefore, when the head is placed on the head cooling pillow, the head is appropriately supported by the support projection while appropriately forming the flow passage by the inner wall of the pillow main body and the partition member.

In the present invention, the head cooling pillow includes, for example, an internally arranged member which is provided with a base part formed in a substantially flat plate shape, the partition member and a plurality of the support projections, and the internally arranged member is disposed in an inside of the pillow main body, and the partition member and a plurality of the support projections are integrally formed with the base part in one piece so as to be stood up from the base part. In this case, handling of the partition member and a plurality of the support projections is easy at the time of assembling of the head cooling pillow.

In the present invention, it is preferable that the partition member is formed in a thin plate shape, the support projection is formed in a substantially cylindrical shape or a substantially circular truncated cone shape and, when viewed in a thickness direction of the head cooling pillow, some support projections of a plurality of the support projections are partly overlapped with the partition member. In other words, some of a plurality of the support projections are integrally formed with the partition member. According to this structure, tilting of the partition member which is formed in a thin plate shape is prevented by the support projection formed in a substantially cylindrical shape or a substantially circular truncated cone shape.

In the present invention, for example, the pillow main body includes a sheet-shaped member which structures a front face or a rear face of the pillow main body, and the partition member and a plurality of the support projections are integrally formed with the sheet-shaped member in one piece so as to be stood up from the sheet-shaped member. In this case, handling of the partition member and a plurality of the support projections is easy at the time of assembling of the head cooling pillow. Further, in this case, in comparison with a case that an internally arranged member is disposed in the inside of the pillow main body, the number of part items of the head cooling pillow is reduced.

In accordance with the present invention, a plurality of the support projections may be disposed on an outer side of the pillow main body. In this case, the pillow main body and the support projections are easily separated from each other. Therefore, for example, when the pillow main body is damaged, only the pillow main body and the partition member disposed in the inside of the pillow main body are easily exchanged. Further, when the support projections are damaged, only the support projections are easily exchanged.

In the present invention, it may be structured that the support member is structured of a plurality of plate-shaped members which are disposed in the flow passage in the inside of the pillow main body, a plurality of the plate-shaped members is superposed on each other in a thickness direction of the head cooling pillow, and the plate-shaped member is formed with a plurality of passing holes so that the refrigerant is passed through the flow passage. In this case, the head is supported by the support member which is structured of the plate-shaped member. Therefore, an area of a portion of the support member which supports the head is increased. Accordingly, placing feeling of the head on the head cooling pillow is improved effectively.

In the present invention, it is preferable that the passing hole is formed so as to penetrate through the plate-shaped member in the thickness direction of the head cooling pillow and, when one of the plate-shaped members adjacent to each other in the thickness direction of the head cooling pillow is a first plate-shaped member and the other of the plate-shaped members is a second plate-shaped member and, when the head cooling pillow is viewed in the thickness direction, a center of the passing hole formed in the first plate-shaped member and a center of the passing hole formed in the second plate-shaped member are displaced from each other, and the passing hole which is formed in the first plate-shaped member is partly overlapped with at least two pieces of the passing holes formed in the second plate-shaped member, and the passing hole which is formed in the second plate-shaped member is partly overlapped with at least two pieces of the passing holes formed in the first plate-shaped member. According to this structure, an area of a portion of the support member which supports the head is increased with a relatively simple structure while securing the flow rate of refrigerant passing through the flow passage.

The head cooling pillow in accordance with the present invention may be used in a head cooling device including a pump which is connected to the head cooling pillow and is structured to supply the refrigerant to the head cooling pillow, and a cooler which cools the refrigerant supplied to the head cooling pillow. In the head cooling device, cooling efficiency for the head is enhanced, the pressure rise in the inside of the head cooling pillow is suppressed, and placing feeling of the head on the head cooling pillow is improved.

In the present invention, it is preferable that the head cooling pillow, the pump and the cooler are connected with each other without a tank, and a closed loop is formed by the head cooling pillow, the pump and the cooler. According to this structure, the actual pump head of the pump is zero and thus the pump having a small capacity can be used. Therefore, the size of the pump is reduced.

### [Advantageous Effects of Invention]

As described above, according to the head cooling pillow and the head cooling device in accordance with the present invention, cooling efficiency for the head is enhanced, the pressure rise in the inside of the head cooling pillow is suppressed, and placing feeling of the head on the head cooling pillow is improved.

### [Brief Description of Drawings]

[Fig. 1]

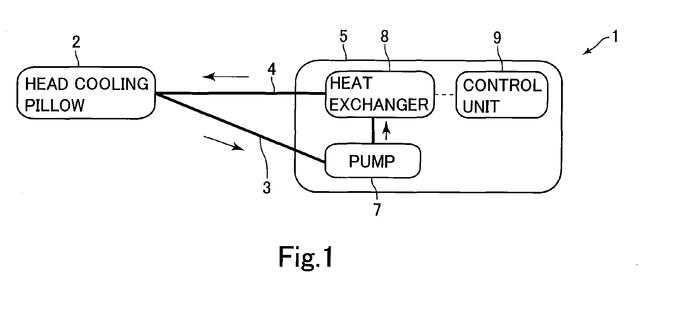

   Fig. 1 is a block diagram showing a schematic structure of a head cooling device in accordance with an embodiment of the present invention.
[Fig. 2]

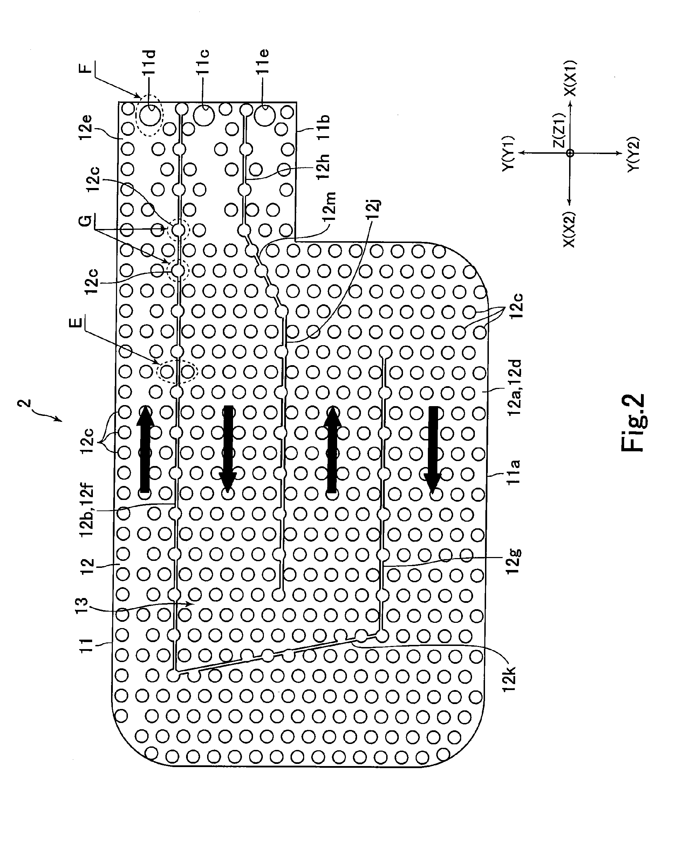

   Fig. 2 is a plan view showing a schematic structure of a head cooling pillow shown in Fig. 1.
[Fig. 3]

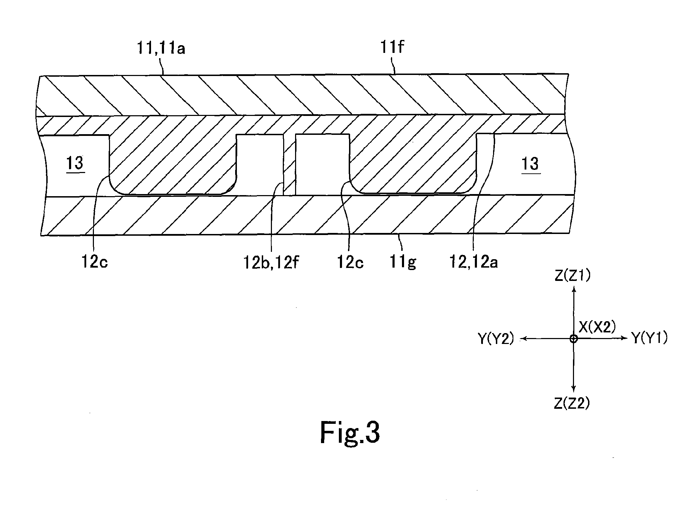

   Fig. 3 is a cross-sectional view showing a cross section of the "E" part in Fig. 2.
[Fig. 4]

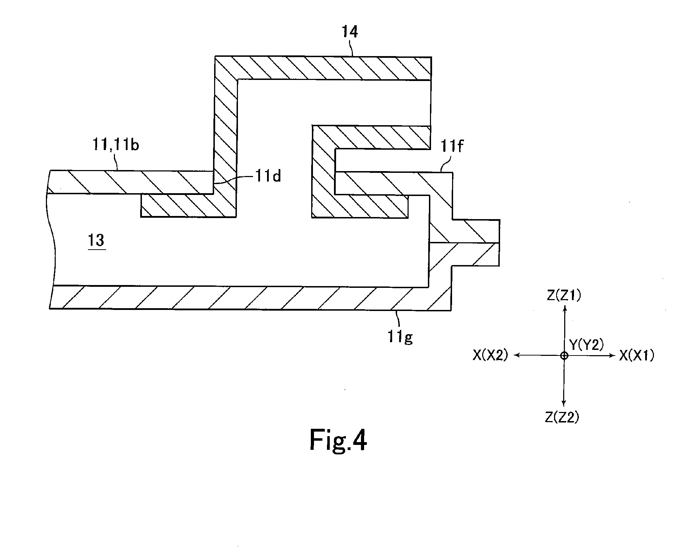

   Fig. 4 is a cross-sectional view showing a cross section of the "F" part in Fig. 2.
[Fig. 5]

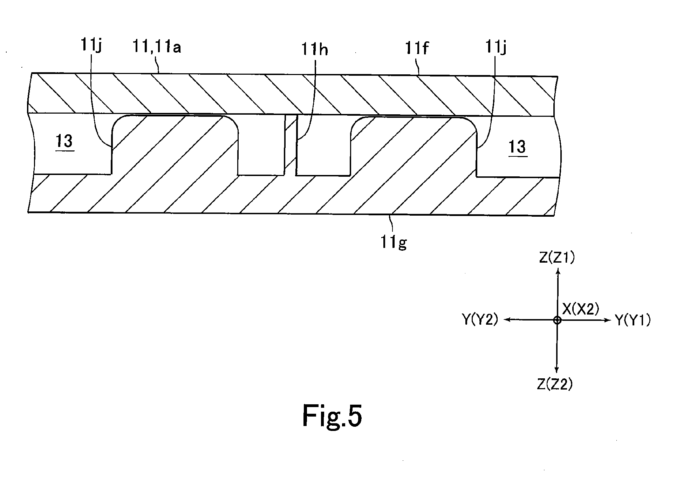

   Fig. 5 is an explanatory cross-sectional view showing a partition part and a support projection in accordance with another embodiment of the present invention.
[Fig. 6]

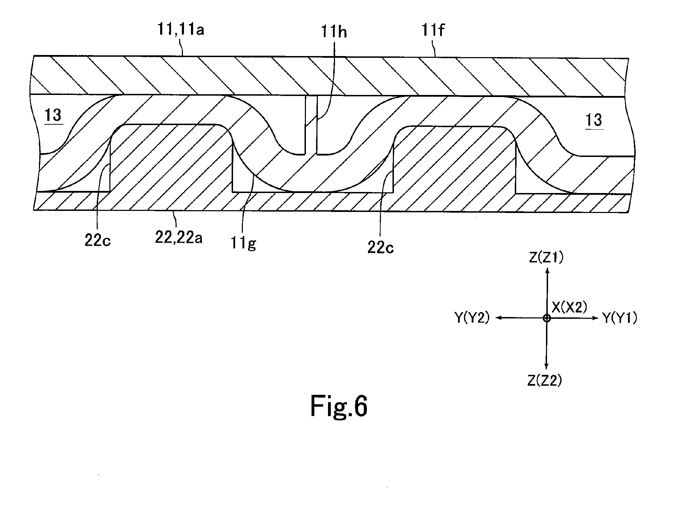

   Fig. 6 is an explanatory cross-sectional view showing a partition part and a support projection in accordance with another embodiment of the present invention.
[Fig. 7]

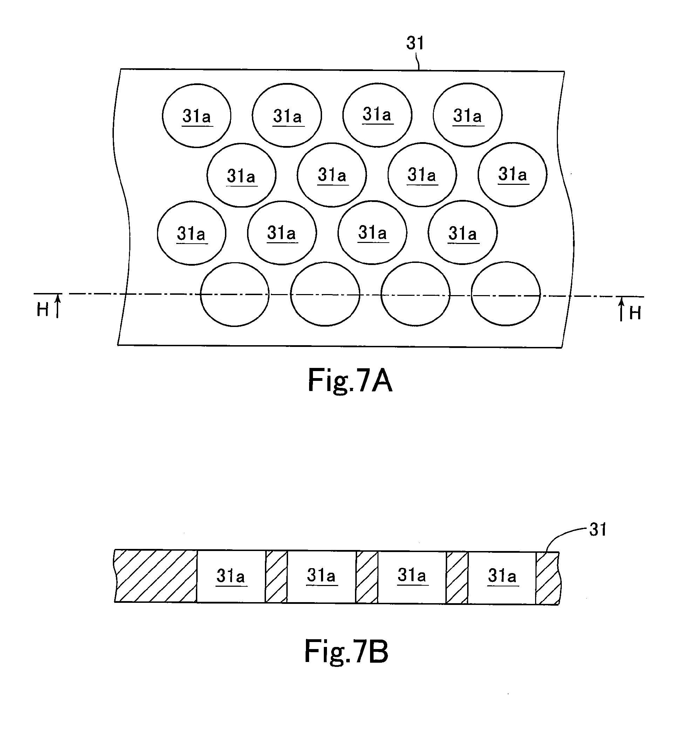

   Figs. 7(A) and 7(B) are views showing a part of a plate-shaped member structuring a support member in accordance with another embodiment of the present invention. Fig. 7(A) is its plan view and Fig. 7(B) is a cross-sectional view showing the "H-H" cross section in Fig. 7(A).
[Fig. 8]

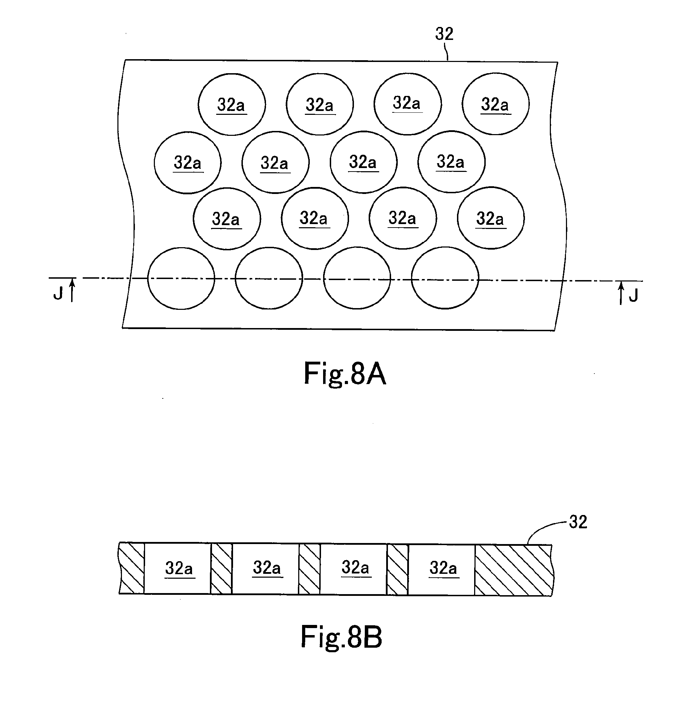

   Figs. 8(A) and 8(B) are views showing a part of a plate-shaped member structuring a support member in accordance with another embodiment of the present invention. Fig. 8(A) is its plan view and Fig. 8(B) is a cross-sectional view showing the "J-J" cross section in Fig. 8(A).
[Fig. 9]

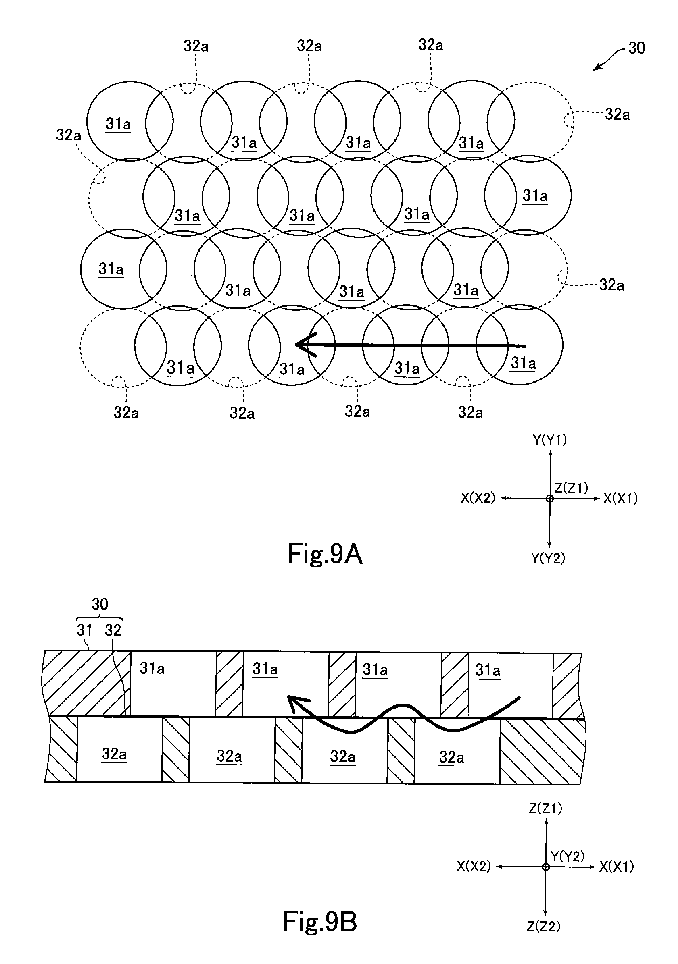

   Figs. 9(A) and 9(B) are views showing a state that the plate-shaped member shown in Figs. 7(A) and 7(B) and the plate-shaped member shown in Figs. 8(A) and 8(B) are superposed on each other. Fig. 9(A) is its plan view and Fig. 9(B) is its cross-sectional view.
[Fig. 10]

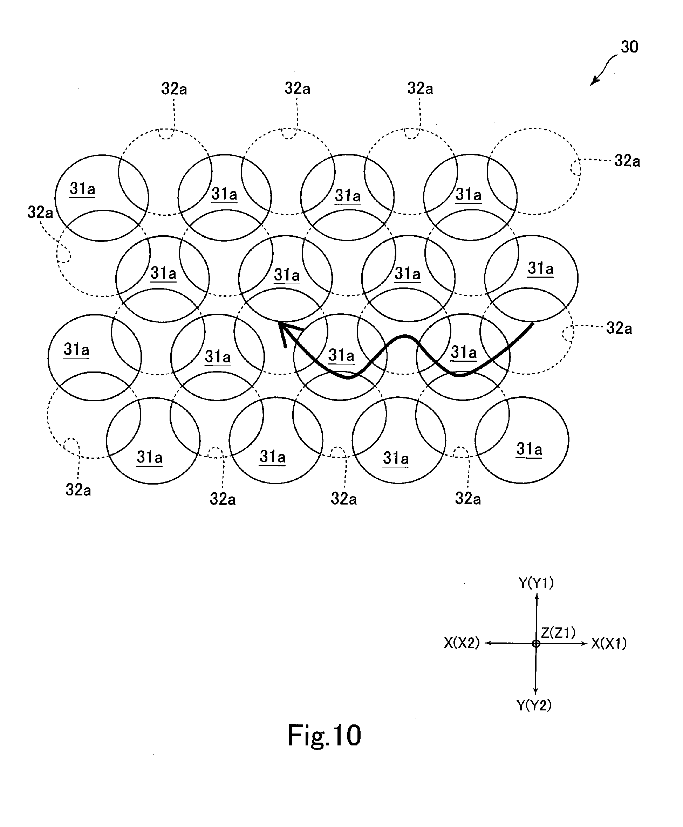

   Fig. 10 is a plan view showing another example in a state that the plate-shaped member shown in Figs. 7(A) and 7(B) and the plate-shaped member shown in Figs. 8(A) and 8(B) are superposed on each other.

### [Description of Embodiments]

An embodiment of the present invention will be described below with reference to the accompanying drawings.

### (Schematic Structure of Head Cooling Device)

Fig. 1 is a block diagram showing a schematic structure of a head cooling device 1 in accordance with an embodiment of the present invention.

The head cooling device 1 in this embodiment is a device structured to cool the head of a lying user and, for example, is set and used in a bed in a hospital, a care facility or the like. The head cooling device 1 includes, as shown in Fig. 1, a head cooling pillow 2 (hereinafter, referred to as "pillow 2") on which the head of a user is placed and a mechanical part 5 connected with the pillow 2 through tubes 3 and 4 having flexibility. Refrigerant such as water is circulated between the pillow 2 and the mechanical part 5. Further, the pillow 2 is, for example, set on the bed and the mechanical part 5 is set to a fence surrounding the bed. Since the pillow 2 is formed in a flat shape as described below, the pillow 2 is normally used in a state that the pillow 2 is superposed on a normal pillow or cushion. In other words, the height of the pillow 2 on which the head of a user is placed is adjusted by using a normal pillow or cushion.

The tubes 3 and 4 are formed of heat insulation material which is excellent in heat insulation property and is formed of material which is hard to be folded. The mechanical part 5 includes a pump 7 structured to supply refrigerant to the pillow 2, a heat exchanger 8 as a cooler which cools the refrigerant supplied to the pillow 2, and a control unit 9 which controls the heat exchanger 8. The pump 7 is connected with a refrigerant discharge side of the pillow 2 through the tube 3. The heat exchanger 8 is connected with a refrigerant supply side of the pillow 2 through the tube 4. Further, the heat exchanger 8 is connected with a discharge side of the pump 7 through a predetermined pipe. The heat exchanger 8 includes, for example, a thermoelectric element such as a Peltier element and cools the refrigerant supplied to the pillow 2 by the pump 7. The control unit 9 controls the heat exchanger 8 to control the temperature of the refrigerant supplied to the pillow 2.

In this embodiment, the pillow 2, the pump 7 and the heat exchanger 8 are connected with each other without using a tank in which the refrigerant is stored. In other words, in this embodiment, a closed loop is formed by the pillow 2, the pump 7 and the heat exchanger 8. In accordance with an embodiment of the present invention, the mechanical part 5 may include a small tank for bleeding of air in the refrigerant which is circulated.

### (Structure of Head Cooling Pillow)

Fig. 2 is a plan view showing a schematic structure of the head cooling pillow 2 shown in Fig. 1. Fig. 3 is a cross-sectional view showing a cross section of the "E" part in Fig. 2. Fig. 4 is a cross-sectional view showing a cross section of the "F" part in Fig. 2. In the following descriptions, respective three directions perpendicular to each other are set to be an "X" direction, a "Y" direction and a "Z" direction and the "X" direction is set to be a right and left direction, the "Y" direction is a front and rear direction, and the "Z" direction is an upper and lower direction. Further, an "X1" direction side is set to be a "right" side, an "X2" direction side is a "left" side, a "Y1" direction side is a "rear" side, a "Y2" direction side is a "front" side, a "Z1" direction side is an "upper" side, and a "Z2" direction side is a "lower" side. In Fig. 3, a cross section of the "E" part is shown which is parallel to a plane structured of the front and rear direction and the upper and lower direction and, in Fig. 4, a cross section of the "F" part is shown which is parallel to a plane structured of the right and left direction and the upper and lower direction.

The pillow 2 is formed in a flat shape in which the upper and lower direction is its flattened direction. In other words, the upper and lower direction is a thickness direction of the pillow 2. The pillow 2 includes a pillow main body 11 which is formed in a bag shape and a spacer 12 as an internally arranged member which is disposed in an inside of the pillow main body 11. In this embodiment, the head which is supported by the pillow 2 is abutted with an upper face of the pillow 2. Further, the right and left direction is substantially coincided with a widthwise direction of the head which is supported by the pillow 2 and the body of a user whose head is supported by the pillow 2 is disposed on a front side ("Y2" direction side).

The pillow main body 11 is formed of relatively flexible resin, hard rubber or the like. The pillow main body 11 in this embodiment is formed of thermoplastic urethane (TPU) in consideration of its strength. The pillow main body 11 is formed by joining an upper face part 11f in a sheet shape, which structures an upper face (front face) of the pillow main body 11, to an under face part 11g in a sheet shape which structures an under face (back face) of the pillow main body 11. Further, the pillow main body 11 is, as shown in Fig. 2, provided with a rectangular parallelepiped part 11a formed in a flat and roughly rectangular parallelepiped shape and a protruded part 11b in a flat and substantially rectangular parallelepiped shape which is protruded from a right rear end side of the rectangular parallelepiped part 11a to the right direction. The pillow main body 11 is sealed up so that the refrigerant in its inside is not leaked out from a portion except a supply port 11c, a discharge port 11d and an air bleeding port 11e described below. Specifically, an outer peripheral end of the upper face part 11f and an outer peripheral end of the under face part 11g are joined to each other over their whole peripheries so that the refrigerant in the inside is not leaked out from a portion except the supply port 11c, the discharge port 11d and the air bleeding port 11e described below.

The spacer 12 is formed of relatively flexible resin, hard rubber or the like. Specifically, the spacer 12 is formed of resin having flexibility, hard rubber or the like so as to be capable of following the shape of the head (capable of fitting the shape of the head) to some extent. For example, the spacer 12 is formed of polypropylene, polyethylene or hard silicone rubber. The spacer 12 is provided with a base part 12a which is formed in a substantially flat plate shape, a partition part 12b as a partition member for forming a flow passage 13 through which the refrigerant is passed, and a plurality of support projections 12c as a support member for supporting the head. The partition part 12b and the support projection 12c are, as shown in Fig. 3, integrally formed with the base part 12a in one piece so as to protrude from the under face of the base part 12a to a lower side. In accordance with an embodiment of the present invention, the spacer 12 may be formed of TPU similarly to the pillow main body 11.

The base part 12a is structured of a first base part 12d, which is disposed in the inside of the rectangular parallelepiped part 11a of the pillow main body 11, and a second base part 12e which is protruded to the right direction from a right rear end side of the first base part 12d and is disposed in the inside of the protruded part 11b. The shape of the first base part 12d when viewed in the upper and lower direction is formed in a roughly rectangular shape which is substantially the same shape as the shape of the rectangular parallelepiped part 11a. The shape of the second base part 12e when viewed in the upper and lower direction is formed in a substantially rectangular shape which is substantially the same shape as the shape of the protruded part 11b.

The partition part 12b is formed in a thin plate shape whose thickness is substantially constant. Further, the partition part 12b is structured of partition plates 12f, 12g, 12h and 12j, which are substantially parallel to the right and left direction and the upper and lower direction, and partition plates 12k and 12m which are substantially parallel to the upper and lower direction and are inclined with respect to the front and rear direction and the right and left direction. The partition plate 12f is formed on the rear end side of the base part 12a from the right end of the second base part 12e to a left end side of the first base part 12d. The partition plate 12g is formed on the front end side of the base part 12a from the right end side to the left end side of the first base part 12d. A left end of the partition plate 12f and a left end of the partition plate 12g are connected with each other through the partition plate 12k. The partition plates 12h and 12j are disposed from the rear side in this order in the front and rear direction between the partition plate 12f and the partition plate 12g. Further, the partition plate 12h is formed from the right end of the second base part 12e to the left end side of the second base part 12e, and the partition plate 12j is formed from the right end side of the first base part 12d to the left end side of the first base part 12d. The left end of the partition plate 12h and the right end of the partition plate 12j are connected with each other through the partition plate 12m. Further, a space is formed between the left end of the partition plate 12j and the partition plate 12k in the right and left direction.

A supply port 11c for supplying refrigerant to the flow passage 13, a discharge port 11d for discharging the refrigerant from the flow passage 13, and an air bleeding port 11e for bleeding air in the inside of the flow passage 13 at an intermediate position of the flow passage 13 are formed in the vicinity of the right end of the protruded part 11b so as to be adjacent to each other in the front and rear direction. In the front and rear direction, the discharge port 11d is formed between the rear end of the second base part 12e and the partition plate 12f, the supply port 11c is formed between the partition plate 12f and the partition plate 12h, and the air bleeding port 11e is formed between the partition plate 12h and the front end of the second base part 12e. As shown in Fig. 4, a joint 14 is fixed to the discharge port 11d and the tube 3 is connected with the joint 14. Similarly, a joint 14 is fixed to the supply port 11c and the tube 4 is connected with the joint 14. Further, a joint 14 is fixed to the air bleeding port 11e and the joint 14 is normally closed with a plug. A safe cover is attached to the joints 14 for preventing an accident to secure safety of a user.

In this embodiment, the flow passage 13 is formed by the base part 12a and the partition part 12b in the inside of the pillow main body 11 so that the refrigerant supplied from the supply port 11c is passed through between the partition plate 12f and the partition plate 12h, between the partition plate 12f and the partition plate 12m, between the partition plate 12f and the partition plate 12j, between the partition plate 12j and the partition plate 12g, between the partition plate 12g and the front end of the pillow main body 11, between the partition plate 12k and the left end of the pillow main body 11, and between the partition plate 12f and the rear end of the pillow main body 11 in this order and is discharged from the discharge port 11d. In other words, the inside of the pillow main body 11 is formed with the flow passage 13 in which the refrigerant goes and returns two times between the right end side and the left end side of the pillow main body 11. In this embodiment, the head supported by the pillow 2 is cooled by the refrigerant passing through the flow passage 13.

A plurality of the support projections 12c is formed in the inside of the flow passage 13. In this embodiment, a large number of the support projections 12c is formed with a substantially equal interval over the entire region of the first base part 12d. A plurality of the support projections 12c which are formed in the first base part 12d is disposed in a zigzag manner. Further, a number of the support projections 12c is formed over the substantially entire region of the second base part 12e with an interval wider than the interval of the support projections 12c formed in the first base part 12d. The support projection 12c is formed in a substantially cylindrical shape. A diameter of the support projection 12c is wider than a width of the partition part 12b. As shown in Fig. 3, "Rounding" work (curved face working) is performed on a boundary portion between the side face and the under face of the support projection 12c. Further, when viewed in the upper and lower direction, some support projections 12c are, for example, as shown by the "G" part in Fig. 2, partly overlapped with the partition part 12b. In other words, some support projections 12c are integrally formed with the partition part 12b. In this embodiment, the partition plates 12f through 12h and the partition plates 12j and 12m except the partition plate 12k are overlapped with a plurality of the support projections 12c with a substantially equal interval when viewed in the upper and lower direction.

In this embodiment, as shown in Fig. 3, a height of the support projection 12c and a height of the partition part 12b are set to be substantially equal to each other in the upper and lower direction. Therefore, in a state that the head is not placed on the pillow 2, an upper face of the base part 12a and an under face of the upper face part 11f of the pillow main body 11 are lightly abutted with each other and under faces of the support projection 12c and the partition part 12b and an upper face of the under face part 11g of the pillow main body 11 are lightly abutted with each other. Further, when the head is placed on the pillow 2, the upper face of the base part 12a and the under face of the upper face part 11f are abutted with each other with a certain pressure, and the under faces of the support projection 12c and the partition part 12b and the upper face of the under face part 11g are abutted with each other with a certain pressure. Further, the refrigerant flowing through the flow passage 13 is passed through between the support projections 12c.

In accordance with an embodiment of the present invention, in order to surely prevent the refrigerant from flowing into a space between the upper face of the base part 12a and the under face of the upper face part 11f of the pillow main body 11, the upper face of the base part 12a may be fixed to the under face of the upper face part 11f by an adhesive or the like. In this case, the entire upper face of the base part 12a may be joined to the under face of the upper face part 11f by an adhesive or the like, or the upper face of the base part 12a may be partly joined to the under face of the upper face part 11f by an adhesive or the like.

(Principal Effects in this Embodiment)
As described above, in this embodiment, the head supported by the pillow 2 is cooled by the refrigerant passing through the flow passage 13 which is formed in the inside of the pillow main body 11. Therefore, in this embodiment, the head is cooled by one time of heat exchange and, as a result, cooling efficiency for the head is enhanced.

Further, in this embodiment, a plurality of the support projections 12c is provided in addition to the partition part 12b disposed in the inside of the pillow main body 11 and thus, when the head is placed on the pillow 2, the head is supported by a plurality of the support projections 12c and the partition part 12b through the base part 12a. Therefore, in this embodiment, the rise of the internal pressure of the pillow 2 (pressure in the inside of the flow passage 13) is restrained. Especially, in this embodiment, a plurality of the support projections 12c is disposed in the inside of the pillow main body 11. Therefore, in comparison with a case that a plurality of the support projections 12c is disposed on an outer side of the pillow main body 11, the rise of the internal pressure of the pillow 2 is effectively suppressed.

Further, in this embodiment, a plurality of the support projections 12c is provided and thus the head placed on the pillow 2 is also supported by a plurality of the support projections 12c in addition to the partition part 12b through the base part 12a. Therefore, in this embodiment, placing feeling of the head on the pillow 2 is improved.

In this embodiment, the height of the partition part 12b and the height of the support projection 12c are set to be substantially equal to each other. Therefore, as described above, when the head is placed on the pillow 2, the upper face of the base part 12a and the under face of the upper face part 11f of the pillow main body 11 are abutted with each other with a certain pressure, and the under faces of the support projections 12c and the partition part 12b and the upper face of the under face part 11g of the pillow main body 11 are abutted with each other with a certain pressure. Accordingly, when the head is placed on the pillow 2, the head is appropriately supported by the support projections 12c through the base part 12a while appropriately forming the flow passage 13 by the upper face of the under face part 11g and the partition part 12b.

In this embodiment, the partition part 12b and a plurality of the support projections 12c are integrally formed in one piece with the base part 12a. Therefore, handling of the partition part 12b and a plurality of the support projections 12c is easy at the time of assembling of the pillow 2. Further, in this embodiment, some support projections 12c are integrally formed with the partition part 12b and thus tilting of the partition part 12c which is formed in a thin plate shape is prevented by the support projection 12c that is formed in a substantially cylindrical shape.

In this embodiment, the air bleeding port 11e is formed for bleeding air existed in the inside of the flow passage 13 at an intermediate position of the flow passage 13. Therefore, even when the relatively long flow passage 13 is formed so that the refrigerant goes and returns two times between the right end side and the left end side of the pillow main body 11, air in the inside of the flow passage 13 is easily bled out by utilizing the air bleeding port 11e at the time of filling the refrigerant into the inside of the pillow main body 11. Further, at the time of maintenance or the like of the pillow 2, the refrigerant in the inside of the flow passage 13 is easily removed by utilizing the air bleeding port 11e.

In this embodiment, the pillow 2, the pump 7 and the heat exchanger 8 are connected with each other without using a tank and thus a closed loop is formed by the pillow 2, the pump 7 and the heat exchanger 8. Therefore, the actual pump head of the pump 7 is zero. Accordingly, in this embodiment, the pump 7 having a small capacity can be used and the size of the pump 7 is reduced. Further, in this embodiment, the pillow 2 is formed in a flat shape and thus an amount of the refrigerant required in the head cooling device 1 is reduced.

### (Other Embodiments)

In the embodiment described above, the spacer 12 is disposed so that the upper face of the base part 12a is abutted with the under face of the upper face part 11f of the pillow main body 11 with which the head is abutted. However, the spacer 12 may be disposed so that the support projections 12c and the partition part 12b are abutted with the under face of the upper face part 11f and the base part 12a is abutted with the upper face of the under face part 11g of the pillow main body 11. In the former case, since the head is supported by the support projections 12c through the base part 12a, in comparison with a case that the head is supported by the support projections 12c without the base part 12a, placing feeling of the head on the pillow 2 is enhanced. On the other hand, in the latter case, the refrigerant is directly contacted with the under face of the upper face part 11f of the pillow main body 11 and thus cooling effect for the head is enhanced.

In the embodiment described above, the partition part 12b and the support projections 12c are integrally formed in one piece through the base part 12a. However, it may be structured that the partition part 12b and the support projections 12c are separately formed from each other and are disposed in the inside of the pillow main body 11. Further, in the embodiment described above, when viewed in the upper and lower direction, some support projections 12c are partly overlapped with the partition part 12b. However, all support projections 12c may be formed so as not to overlap with the partition part 12b. Further, in the embodiment described above, a number of the support projections 12c is formed in the second base part 12e but no support projection 12c may be formed in the second base part 12e.

In the embodiment described above, the support projection 12c is formed in a substantially cylindrical shape but the support projection 12c may be formed in a substantially circular truncated cone shape whose diameter becomes gradually larger toward the upper side. Further, the support projection 12c may be formed in a polygonal pillar shape such as a rectangular pillar shape or may be formed in a polygonal truncated pyramid shape such as a quadrangular truncated pyramid shape. Further, in the embodiment described above, the partition part 12b is formed in a thin plate shape whose thickness is substantially constant but the partition part 12b may be formed so that its cross-sectional shape is a trapezoid shape. In this case, the cross-sectional shape of the partition part 12b is, for example, an isosceles trapezoid shape whose width is gradually widened toward the upper side.

### (First Modified Example of Partition Member and Support Member)

Fig. 5 is an explanatory cross-sectional view showing a partition part 11h and a support projection 11j in accordance with another embodiment of the present invention.

In the embodiment described above, the spacer 12 disposed in the inside of the pillow main body 11 is formed with the partition part 12b as a partition member for forming the flow passage 13 through which the refrigerant is passed and a plurality of the support projections 12c as a support member for supporting the head. However, the present invention is not limited to this embodiment. For example, as shown in Fig. 5, an under face part 11g structuring the under face of the pillow main body 11 may be formed with a partition part 11h as the partition member and a plurality of support projections 11j as the support member. In this case, the partition part 11h and the support projections 11j are formed so as to stand up from an upper face of the under face part 11g to an upper side. Further, for example, the partition part 11h is formed similarly to the partition part 12b and the support projection 11j is formed similarly to the support projection 12c.

In this case, handling of the partition part 11h and a plurality of the support projections 11j is easy at the time of assembling of the pillow 2. Further, in this case, since the spacer 12 is not required to be disposed in the inside of the pillow main body 11, the number of part items of the pillow 2 is reduced and the structure of the pillow 2 is simplified. In accordance with an embodiment of the present invention, a partition part and a plurality of support projections may be formed on the upper face part 11f structuring the upper face of the pillow main body 11. In a case that the partition part 11h and a plurality of the support projections 11j are formed in the under face part 11g, the under face part 11g is a sheet-shaped member on which the partition part 11h and a plurality of the support projections 11j are formed. Further, in a case that the partition part and a plurality of the support projections are formed on the upper face part 11f, the upper face part 11f is a sheet-shaped member on which the partition part and a plurality of the support projections are formed.

### (Second Modified Example of Partition Member and Support Member)

Fig. 6 is an explanatory cross-sectional view showing a partition part 11h and a support projection 22c in accordance with another embodiment of the present invention.

In the embodiment described above, the spacer 12 disposed in the inside of the pillow main body 11 is formed with the partition part 12b as the partition member and a plurality of the support projections 12c as the support member. However, the present invention is not limited to this embodiment. For example, as shown in Fig. 6, it may be structured that an under face part 11g of the pillow main body 11 is formed with a partition part 11h similarly to the above-mentioned first modified example and that a spacer 22 which is disposed on an outer side of the pillow main body 11 is formed with a plurality of support projections 22c as the support member. In this case, the spacer 22 is, for example, formed of material similar to the spacer 12. The support projection 22c is formed so as to stand up to an upper side from an upper face of a base part 22a which is formed in a substantially flat plate shape. Further, the support projection 22c is formed similarly to the support projection 12c.

The spacer 22 is disposed on a lower side with respect to the pillow main body 11. Specifically, the spacer 22 is disposed on a lower side with respect to the pillow main body 11 so that the partition part 11h and the support projection 22c are not overlapped with each other in the upper and lower direction. When the head is placed on the pillow 2, the under face part 11g of the pillow main body 11 is deformed depending on the shape of the support projection 22c. In this case, the pillow main body 11 and the spacer 22 can be easily separated from each other. Therefore, for example, when the pillow main body 11 is damaged, only the pillow main body 11 can be exchanged easily. Further, when the spacer 22 is damaged, only the spacer 22 can be exchanged easily.

### (Third Modified Example of Partition Member and Support Member)

Figs. 7(A) and 7(B) are views showing a part of a plate-shaped member 31 structuring a support member 30 in accordance with another embodiment of the present invention. Fig. 7(A) is its plan view and Fig. 7(B) is a cross-sectional view showing the "H-H" cross section in Fig. 7(A). Figs. 8(A) and 8(B) are views showing a part of a plate-shaped member 32 structuring a support member 30 in accordance with another embodiment of the present invention. Fig. 8(A) is its plan view and Fig. 8(B) is a cross-sectional view showing the "J-J" cross section in Fig. 8(A). Figs. 9(A) and 9(B) are views showing a state that the plate-shaped member 31 shown in Figs. 7(A) and 7(B) and the plate-shaped member 32 shown in Figs. 8(A) and 8(B) are superposed on each other. Fig. 9(A) is its plan view and Fig. 9(B) is its cross-sectional view. Fig. 10 is a plan view showing another example in a state that the plate-shaped member 31 shown in Figs. 7(A) and 7(B) and the plate-shaped member 32 shown in Figs. 8(A) and 8(B) are superposed on each other.

In the embodiment described above, the spacer 12 disposed in the inside of the pillow main body 11 is formed with the partition part 12b as the partition member and a plurality of the support projections 12c as the support member. However, the present invention is not limited to this embodiment. For example, it may be structured that a partition part similar to the partition part 11h shown in Figs. 5 and 6 is formed on the under face part 11g of the pillow main body 11 and the support member 30 is disposed in the inside of the flow passage 13 formed by the partition part.

The support member 30 is, as shown in Figs. 9(A) and 9(B), structured of a plate-shaped member 31 as a first plate-shaped member and a plate-shaped member 32 as a second plate-shaped member, i.e., two pieces of the plate-shaped members 31 and 32. The plate-shaped members 31 and 32 are formed in a flat plate shape. Two plate-shaped members 31 and 32 are overlapped with each other in the thickness direction and are fixed to each other. For example, the plate-shaped members 31 and 32 are overlapped with each other from an upper side in this order and are fixed to each other in the state that an under face of the plate-shaped member 31 and an upper face of the plate-shaped member 32 are abutted with each other. The plate-shaped members 31 and 32 are, for example, formed of similar material to the spacer 12. Further, the support member 30 is disposed in the inside of the flow passage 13 so that the thickness direction of the plate-shaped members 31 and 32 is coincided with the upper and lower direction.

The plate-shaped member 31 is, as shown in Figs. 7(A) and 7(B), formed with a plurality of passing holes 31a which penetrate through the plate-shaped member 31 in the thickness direction of the plate-shaped member 31. A plurality of the passing holes 31a is formed in the plate-shaped member 31 with a substantially equal interval. Further, a plurality of the passing holes 31a is disposed in a zigzag manner. The passing hole 31a is, for example, formed in a circular shape when viewed in the upper and lower direction. Similarly, the plate-shaped member 32 is, as shown in Figs. 8(A) and 8(B), formed with a plurality of passing holes 32a which penetrate through the plate-shaped member 32 in the thickness direction of the plate-shaped member 32. A plurality of the passing holes 32a is formed in the plate-shaped member 32 with an interval substantially equal to the interval of a plurality of the passing holes 31a. Further, a plurality of the passing holes 32a is, similarly to a plurality of the passing holes 31a, disposed in a zigzag manner. The passing hole 32a is formed in a similar shape to the passing hole 31a.

The plate-shaped member 31 and the plate-shaped member 32 are, as shown in Fig. 9(A) and 9(B), superposed on each other in the upper and lower direction so that, when viewed in the upper and lower direction, the center of the passing hole 31a and the center of the passing hole 32a are displaced from each other. Further, the plate-shaped member 31 and the plate-shaped member 32 are superposed on each other in the upper and lower direction so that, when viewed in the upper and lower direction, each of a plurality of the passing holes 31a is partly overlapped with at least two passing holes 32a and each of a plurality of the passing holes 32a is partly overlapped with at least two passing holes 31a. For example, as shown in Fig. 9(A), the plate-shaped member 31 and the plate-shaped member 32 are superposed on each other in the upper and lower direction so that an overlapped area of the passing hole 31a with the passing hole 32a in the right and left direction is larger than an overlapped area of the passing hole 31a with the passing hole 32a in the front and rear direction. Therefore, even when the support member 30 is disposed in the flow passage 13, for example, as shown by the arrow in Figs. 9(A) and 9(B), refrigerant in the inside of the flow passage 13 is alternately passed through the passing hole 31a and the passing hole 32a to flow through the flow passage 13 to the left direction. As described above, even when the support member 30 is disposed in the flow passage 13, a plurality of the passing holes 31a and 32a are formed in the plate-shaped members 31 and 32 so that the refrigerant is passed through the inside of the flow passage 13.

In this case, when the head is placed on the pillow 2, the head is supported by a portion of the plate-shaped member 31 where the passing holes 31a are not formed. Therefore, an area of the portion of the support member 30 which supports the head is increased. Accordingly, placing feeling of the head on the pillow 2 is enhanced effectively. Further, in this case, an area of the portion of the support member 30 which supports the head is increased while securing the flow rate of the refrigerant passing through the flow passage 13 with a relatively simple structure where the center of the passing hole 31a and the center of the passing hole 32a are shifted from each other and, in addition, each of a plurality of the passing holes 31a is partly overlapped with at least two passing holes 32a and each of a plurality of the passing hole 32a is partly overlapped with at least two passing holes 31a.

In accordance with an embodiment of the present invention, the plate-shaped member 31 and the plate-shaped member 32 may be superposed on each other so that, when viewed in the upper and lower direction, each of a plurality of the passing holes 31a is partly overlapped with three passing holes 32a and each of a plurality of the passing holes 32a is partly overlapped with three passing holes 31a. In this case, as shown in Fig. 10, it is preferable that the plate-shaped member 31 and the plate-shaped member 32 are superposed on each other so that, when viewed in the upper and lower direction, each of a plurality of the passing holes 31a is overlapped with each of three passing holes 32a with substantially the same area as each other and, in addition, each of a plurality of the passing hole 32a is overlapped with each of three passing holes 31a with substantially the same area as each other. According to this structure, the hardness of the support member 30 in the upper and lower direction is capable of being set substantially uniform over substantially the entire region of the support member 30. Therefore, placing feeling of the head on the pillow 2 is enhanced further effectively. Further, the withstand load of the support member 30 can be set substantially uniform over substantially the entire region of the support member 30.

Further, the shape of the passing holes 31a and 32a when viewed in the upper and lower direction may be formed in a polygonal shape such as a triangular shape or an elliptic shape. Further, the shape of the passing hole 31a and the shape of the passing hole 32a may be different from each other. Further, the support member 30 may be structured three or more pieces of the plate-shaped member. In this case, it is sufficient that the center of a passing hole, which is formed in one of at least two plate-shaped members adjacent to each other in the upper and lower direction of three or more pieces of the plate-shaped member, and the center of a passing hole formed in the other of the plate-shaped members are displaced from each other and, in addition, that each of the passing holes formed in the one of the plate-shaped members is partly overlapped with at least two or more passing holes formed in the other of the plate-shaped members, and each of the passing holes formed in the other of the plate-shaped members is partly overlapped with at least two or more passing holes formed in the one of the plate-shaped members.

### [Reference Signs List]

- 1: head cooling device
- 2: pillow (head cooling pillow)
- 7: pump
- 8: heat exchanger (cooler)
- 11: pillow main body
- 11g: under face part (sheet-shaped member)
- 11h: partition part (partition member)
- 11j: support projection (support member)
- 12: spacer (internally arranged member)
- 12a: base part
- 12b: partition part (partition member)
- 12c: support projection (support member)
- 13: flow passage
- 22c: support projection (support member)
- 30: support member
- 31: plate-shaped member (first plate-shaped member)
- 31a: passing hole
- 32: plate-shaped member (second plate-shaped member)
- 32a: passing hole
- "Z": thickness direction of head cooling pillow

## Claims

1. A head cooling pillow comprising:
a pillow main body which is formed in a bag shape and is structured so that refrigerant is passed through its inside;
a partition member which is disposed in an inside of the pillow main body for forming an flow passage through which the refrigerant is passed; and
a support member structured to support a head of a user;
wherein the head is cooled by the refrigerant passing through the flow passage.

2. The head cooling pillow according to claim 1, wherein the support member is a plurality of support projections.

3. The head cooling pillow according to claim 2, wherein a plurality of the support projections is disposed in the flow passage in the inside of the pillow main body.

4. The head cooling pillow according to claim 3, wherein a height of the partition member and a height of the support projection in a thickness direction of the head cooling pillow are substantially equal to each other.

5. The head cooling pillow according to claim 3 or 4, further comprising an internally arranged member which is provided with a base part formed in a substantially flat plate shape, the partition member and a plurality of the support projections, and the internally arranged member being disposed in an inside of the pillow main body,
wherein the partition member and a plurality of the support projections are integrally formed with the base part in one piece so as to be stood up from the base part.

6. The head cooling pillow according to claim 5, wherein
the partition member is formed in a thin plate shape,
the support projection is formed in a substantially cylindrical shape or a substantially circular truncated cone shape, and
when viewed in a thickness direction of the head cooling pillow, some support projections of a plurality of the support projections are partly overlapped with the partition member.

7. The head cooling pillow according to claim 3 or 4, wherein
the pillow main body comprises a sheet-shaped member which structures a front face or a rear face of the pillow main body, and
the partition member and a plurality of the support projections are integrally formed with the sheet-shaped member in one piece so as to be stood up from the sheet-shaped member.

8. The head cooling pillow according to claim 2, wherein a plurality of the support projections is disposed on an outer side of the pillow main body.

9. The head cooling pillow according to claim 1, wherein
the support member is structured of a plurality of plate-shaped members which are disposed in the flow passage in the inside of the pillow main body,
the plurality of the plate-shaped members is superposed on each other in a thickness direction of the head cooling pillow, and
the plate-shaped member is formed with a plurality of passing holes so that the refrigerant is passed through the flow passage.

10. The head cooling pillow according to claim 9, wherein
the passing hole is formed so as to penetrate through the plate-shaped member in the thickness direction of the head cooling pillow, and
when one of the plate-shaped members adjacent to each other in the thickness direction of the head cooling pillow is a first plate-shaped member and the other of the plate-shaped members is a second plate-shaped member, and when the head cooling pillow is viewed in the thickness direction,
a center of the passing hole formed in the first plate-shaped member and a center of the passing hole formed in the second plate-shaped member are displaced from each other, and
the passing hole which is formed in the first plate-shaped member is partly overlapped with at least two pieces of the passing holes formed in the second plate-shaped member, and
the passing hole which is formed in the second plate-shaped member is partly overlapped with at least two pieces of the passing holes formed in the first plate-shaped member.

11. A head cooling device comprising:
the head cooling pillow defined in claim 1;
a pump which is connected to the head cooling pillow and is structured to supply the refrigerant to the head cooling pillow; and
a cooler which cools the refrigerant supplied to the head cooling pillow.

12. The head cooling device according to claim 11, wherein
the head cooling pillow, the pump and the cooler are connected with each other without a tank, and
a closed loop is formed by the head cooling pillow, the pump and the cooler.
